# EUROPEAN PATENT APPLICATION

(11) **EP 4 623 839 A1**
(43) Date of publication of application: **01.10.2025**
(21) Application number: 23893394.9
(22) Date of filing: 21.09.2023
(51) Int. Cl.: A61B 17/068

(54) **STAPLER AVAILABLE FOR MANUAL OPERATION UPON FAILURE OF ELECTRIC MODE**

(30) Priority: 21.11.2022 CN 202211452627
(71) Applicant: Innolcon Medical Technology (Suzhou) Co., Ltd., Jiangsu 215000 (CN); Innolcon Medical Technology (Hefei) Co., Ltd., Hefei, Anhui 230000 (CN)
(72) Inventor: HUANG, Aiyu, Suzhou, Jiangsu 215000 (CN); WU, Jun, Suzhou, Jiangsu 215000 (CN); LIU, Wei, Suzhou, Jiangsu 215000 (CN); LUO, Wei, Suzhou, Jiangsu 215000 (CN)
(74) Representative: DREISS Patentanwälte PartG mbB
(86) International application number: PCT/CN2023/120224
(87) International publication number: WO 2024/109301

(57) **Abstract**

Disclosed is a stapler that is manually operable in case of failure in electric mode, including a housing, an effector, a firing control assembly, a swing control assembly, a first manual member and a connecting block. The firing control assembly includes a first drive motor and a gear set, the swing control assembly includes a second drive motor and a transmission assembly, the first manual member drives the connecting block to displace radially, and a distal side of the connecting block is provided with a first limit member and a second limit member that synchronously displace radially therewith, the first limit member is configured to cut off power transmission between the first drive motor and the effector through its radial displacement; meanwhile, the second limit member is configured to cut off power transmission between the second drive motor and the effector through radial displacement thereof. According to the present invention, the electric controls of the firing control assembly and the swing control assembly over the effector are synchronously disabled by means of the first manual member in the failure mode, such that the effector can be returned for safe withdrawal, and can be manually operated through the second manual member to complete the operation.

## Description

This disclosure claims priority to Chinese patent application No. 202211452627.1, titled "STAPLER THAT IS MANUALLY OPERABLE IN CASE OF FAILURE IN ELECTRIC MODE " , filed on November 21, 2022 with the China National Intellectual Property Administration, the entire disclosure of which is incorporated herein by reference.

### FIELD

The present disclosure relates to the technical field of medical device, and in particular, to a stapler that is manually operable in case of failure in electric mode.

### BACKGROUND

Staplers are commonly used medical devices that can clamp tissues to perform cutting and stapling. The staplers are mainly divided into manual staplers and electric staplers. The electric stapler obtains power through electrical energy and is widely popularized and used due to its use stability and convenience. In actual, the end effector of the electric stapler is electrically controlled to bend and rotate so as to clamp the tissues to be sutured, and is returned to its neutral position after the cutting and anastomosis are completed and is withdrawn.

However, the electric stapler may fail due to insufficient battery power or motor failure. When such an emergency occurs during surgery, it may be difficult to withdrawn the electric stapler from the patient's body, and the surgery may not be continued. Furthermore, it needs to remove the stapler through open surgery, which not only interrupts the surgery but also causes secondary damage to the patient. Some existing staplers are provided with retraction mechanisms to help to withdraw the stapler after failure, as disclosed in the patent application with publication number CN113796906A. However, such stapler may only ensure safe withdrawal and cannot continue the surgery. In this case, the doctor has to replace the stapler and repeat the surgery once the stapler fails during surgery, which is time-consuming and labor-intensive, increases the patient's pain and surgical costs, and would increase the difficulty of suturing during the second surgery.

Therefore, there is an urgent requirement to ensure the normal operation in case that the stapler is in failure.

### SUMMARY OF THE INVENTION

An object of the present disclosure is to overcome the deficiencies of the prior art and to provide a stapler that is manually operable in case of failure in electric mode.

The object of the present disclosure is achieved through the following technical solutions:
A stapler that is manually operable in case of failure in electric mode includes a housing, an effector arranged at the front end of the housing, a firing control assembly arranged in the housing for driving the effector to perform closing and anastomosing operations, and a swing control assembly for driving the effector to swing. The firing control assembly includes a first drive motor and a gear set, wherein the gear set includes a driving gear arranged on a motor shaft of the first drive motor, and a driven gear configured to mesh with the driving gear and transmit power to the effector. The swing control assembly includes a second drive motor and a transmission assembly, wherein the transmission assembly includes a driving block screwed on a motor shaft of the second drive motor, and a driving rod configured to receive an axial driving force of the driving block via a plug-in connector and transmitting the driving force to the effector. The stapler further includes a first manual member and a connecting block. A proximal side of the connecting block is configured to engage with the first manual member and be driven by the first manual member to displace radially, and a distal side of the connecting block is disposed with a first limit member and a second limit member that displace radially synchronously with the connecting block. The first limit member is configured to release axial limitation on the driven gear by means of radial displacement of the first limit member, such that the driven gear can be forced to displace axially to disengage from the driving gear, thereby cutting off the power transmission between the first drive motor and the effector; at the same time, the second limit member is configured to drive the plug-in connector to move synchronously radially by means of radial displacement of the second limit member to disengage the driving block from the driving rod, thereby cutting off the power transmission between the second drive motor and the effector.

In some embodiments, the first manual member is embedded in an end cover, and its proximal end has a driving part that is configured to be manually driven to rotate. An insertion rod is eccentrically provided on a distal end surface of the first manual member, and the insertion rod is plugged into the proximal side of the connecting block to convert rotational movement of the first manual member into radial movement of the connecting block.

In some embodiments, the firing control assembly also includes a lead screw and a displacement member, wherein the displacement member is threaded on a threaded portion of the lead screw, the displacement member is fixedly connected to a main shaft, and rotation of the lead screw drives the displacement member to move axially, which in turn drives the main shaft to move axially; the driven gear is movably sleeved on the lead screw, and can drive the lead screw to rotate synchronously through a spline arranged at a central portion of the driven gear.

In some embodiments, a spring is sleeved on the lead screw and abuts against a front end surface of the driven gear. The spring always applies a driving force to the driven gear to drive the driven gear to move axially toward a tail end of the lead screw.

In some embodiments, a top portion of the first limit member is fixedly connected to the connecting block, and a bottom portion of the first limit member is configured to match with an outer contour of the lead screw. A fixing block is provided at the tail end of the lead screw. In an initial state, the bottom portion of the first limit member is engaged between a rear end surface of the driven gear and a front end surface of the fixing block to limit axial position of the driven gear such that the driving gear and the driven gear always remain meshed with each other; and in a second state, the first limit member moves radially such that its bottom portion is separated from the driven gear and the fixing block, and at the same time, the driven gear is driven by the spring to move axially and is then separated from the driving gear.

In some embodiments, a second manual member is embedded in the end cover, the second manual member is fixedly connected to the tail end of the lead screw, and rotation of the second manual member can drive the lead screw to rotate synchronously.

In some embodiments, a through hole is provided at a connection of the driving block and the driving rod; a top portion of the plug-in connector is engaged in a top groove of the driving block, a rod portion of the plug-in connector is inserted in the through hole to connect the driving block and the driving rod, and a side of the plug-in connector is connected to the second limit member.

In some embodiments, a bottom end of the side of the plug-in connector has an outwardly protruding foot, and the second limiting member has an elongated groove arranged parallel to the driving rod. The foot is slidably arranged in the elongated groove, such that the plug-in connector can move axially relative to the second limiting member.

In some embodiments, the second limiting member is an elongated extension plate, and a proximal end of the second limiting member is disposed with a driving waist-shaped hole that extends obliquely. A driving pin is fixedly arranged on a side wall of the connecting block, and the driving pin is slidably arranged in the driving waist-shaped hole. Radial movement of the driving pin can drive the second limiting member to move radially synchronously.

In some embodiments, the second limiting member is further provided thereon with at least one driven waist-shaped hole that is arranged perpendicular to the driving waist-shaped hole, and a positioning pin is slidably arranged in the driven waist-shaped hole, and the positioning pin is fixedly arranged on the housing.

The beneficial effects of the present disclosure mainly includes:
The connecting block, the first limit member and the second limit member are connected, and a first manual member is arranged such that rotation of the first manual member drives the connecting block to move radially, such that the connecting block drives the first limit member and the second limit member to move radially synchronously, so as to simultaneously release the power transmissions between the first drive motor, the second drive motor and the effector, and thus the effector can return to its neutral state and be withdrawn safely in the failure mode to ensure safety. At the same time, the second manual member can be rotated to replace the first drive motor to manually operate the effector, such that the effector can continue to complete the anastomosis of tissues in the failure mode, thereby ensuring smooth progress and completion rate of the operation and avoiding repeated actions in a secondary operation.

### BRIEF DESCRIPTION OF THE DRAWINGS

Technical solutions according to the present disclosure are further described below in conjunction with the accompanying drawings, wherein:
FIG. 1 is a front view of an internal structure of an embodiment of the present disclosure in an initial state;
FIG. 2 is a front view of an internal structure of an embodiment of the present disclosure in a failure mode;
FIG. 3 is a schematic diagram of an internal structure of an embodiment of the present disclosure in a failure mode;
FIG. 4 is an enlarged schematic diagram of part A in FIG. 3;
FIG. 5 is a schematic diagram of an internal structure of another side of an embodiment of the present disclosure in an initial state;
FIG. 6 is a front view of an internal structure of an embodiment of the present disclosure in an initial state; and
FIG. 7 is a top view of partial structure of an embodiment of the present disclosure in a failure mode.

### DETAILED DESCRIPTION OF THE EMBODIMENTS

The present disclosure will be described in detail below in conjunction with the embodiments illustrated in the accompanying drawings. However, the present disclosure is not limited to these embodiments, and any structural, methodological, or functional changes made by a person skilled in the art based on these embodiments are all within the protection scope of the present disclosure.

It should be noted that, orientations or positional relationships indicated by terms such as "center", "up", "down", "left", "right", "front", "back", "vertical", "horizontal", "inner", "outer", "proximal", "distal" and the like used in descriptions of the technical solutions are based on orientations or positional relationships depicted in the drawings, and are only for the convenience of description and simplify the description, rather than indicating or implying that the device or element referred to must have specific orientation, or be constructed and operated in specific orientation, and therefore cannot be understood as limitations to the present disclosure. In addition, terms such as "first", "second" and "third" are used for descriptive purposes only, and shall not be understood as indicating or implying relative importance. Moreover, in the description of the technical solutions, with an operator as a reference, a direction close to the operator is proximal, and a direction away from the operator is distal.

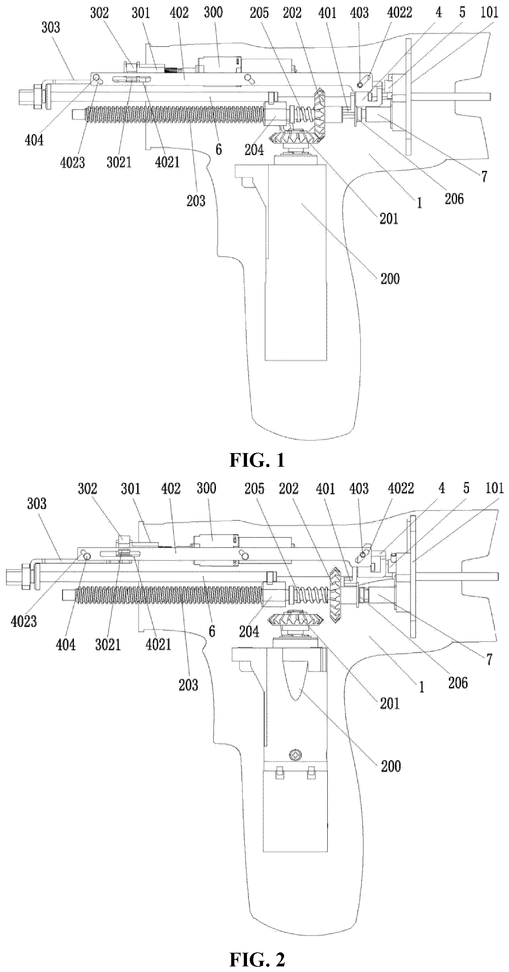

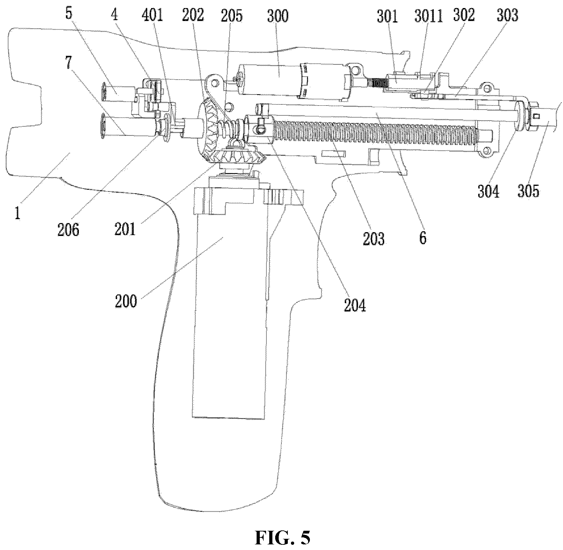

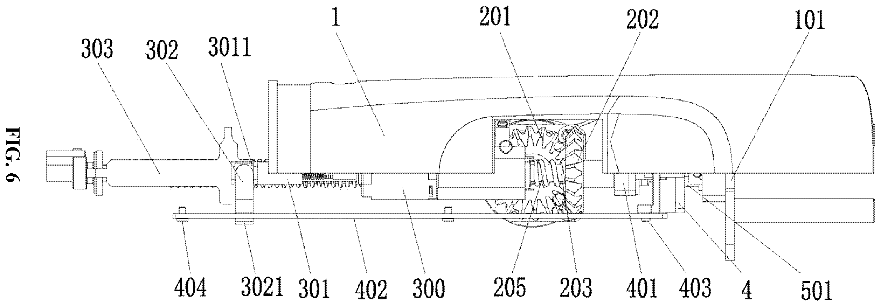

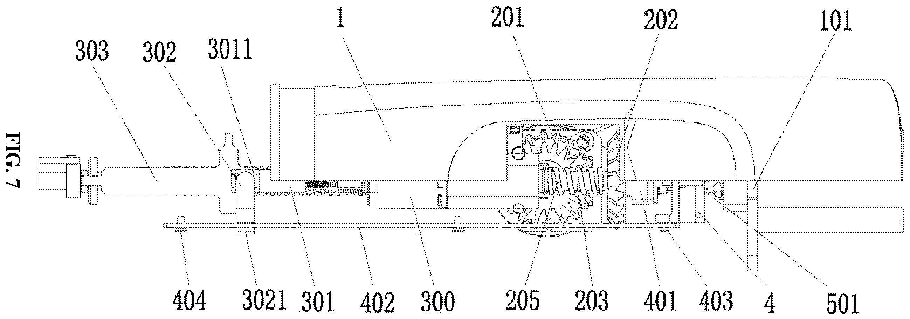

As shown in FIGS. 1 to 7, the present disclosure discloses a stapler that is manually operable in case of failure in an electric mode. The stapler includes a housing 1, an effector disposed at a front end of the housing 1, a firing control assembly arranged in the housing 1 for driving the effector to perform closing and stapling operations, and a swing control assembly for driving the effector to swing. The firing control assembly includes a first drive motor 200 and a gear set. The gear set includes a driving gear 201 disposed on a motor shaft of the first drive motor 200, and a driven gear 202 that is configured to mesh with the driving gear 201 and transmit power to the effector. The swing control assembly includes a second drive motor 300 and a transmission assembly, wherein the transmission assembly includes a driving block 301 connected on a motor shaft of the second drive motor 300 in a threaded manner, and a driving rod 303 that is configured to receive an axial driving force from the driving block 301 through a plug-in connector 302 and transmit the driving force to the effector. The stapler further includes a first manual member 5 and a connecting block 4. A proximal side of the connecting block 4 is configured to cooperate with the first manual member 5 and is driven by the first manual member 5 to displace radially, and a distal side of the connecting block 4 is disposed with a first limit member 401 and a second limit member 402 which are radially displacable synchronously with the connecting block. Radial displacement of the first limit member 401 can release axial limitation on the driven gear 202, such that the driven gear 202 is displaced axially under a force so as to be disengaged from the driving gear 201, thereby cutting off the power transmission between the first drive motor 200 and the effector; at the same time, radial displacement of the second limit member 402 drives the plug-in connector 302 to move synchronously radially to disengage the driving block 301 from the driving rod 303, thereby cutting off the power transmission between the second drive motor 300 and the effector.

The effector in this solution includes a jaw, a staple cartridge seat, an anvil, a steering tab, a blade, an actuating sleeve and other component that is used to perform cutting and stapling functions. These components are available from the prior art, which are not focus of this disclosure, and thus will not be elaborated herein.

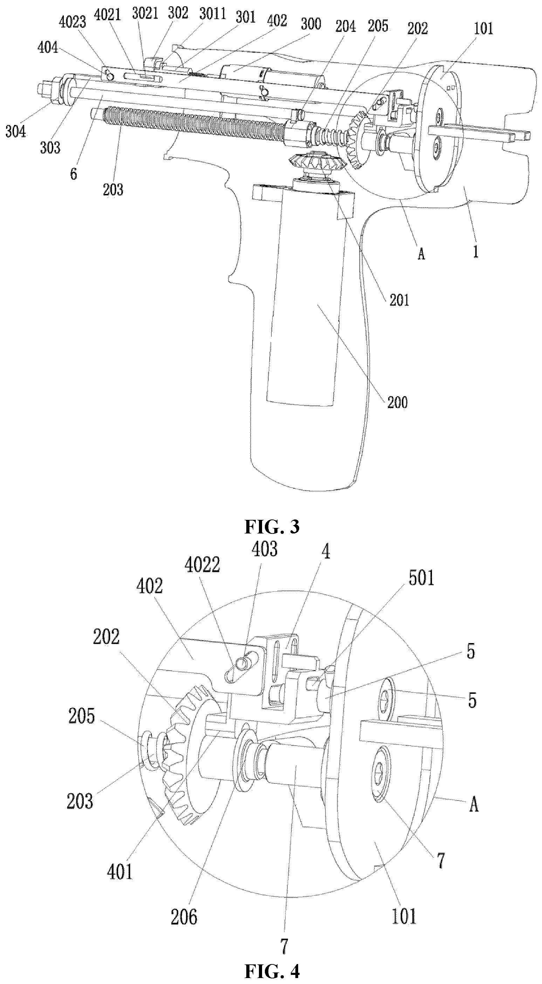

As shown in FIGS. 3 and 4, the first manual member 5 is embedded in an end cover 101. A proximal end of the first manual member has a driving part that is configured to manually drive the first manual member to rotate, and an insertion rod 501 is eccentrically arranged on a distal end surface of the first manual member. The insertion rod 501 is plugged into the proximal side of the connecting block 4 to convert the rotational movement of the first manual member 5 into the radial movement of the connecting block 4. In some feasible embodiments, the first manual member 5 may be a rotatable post and does not protrude out of the end cover 101. The space between the end cover 101 and the housing 1 is used to arrange a battery. In normal state of the battery, the first manual member 5 is shielded by the battery to avoid manual accidental touch. The first manual member 5 is embedded in the end cover 101, which may keep the position of the first manual member 5 relatively fixed, and will not affect the use of the stapler in its normal state. The driving part is configured as a recess formed on the distal end surface of the first manual member 5, and an inner wall of the recess is non-circular. In some feasible embodiments, the inner wall of the recess may be hexagonal to facilitate rotation of a plug-in tool. In another feasible embodiment, the first manual member 5 may also adopt other feasible movement modes to drive the connecting block 4 to move radially, for example, the end cover 101 may be provided thereon with a vertical slide groove such that the first manual member 5 can be directly radially moved to drive the connecting block 4 to move radially synchronously with first manual member.

The firing control assembly also includes a lead screw 203 and a displacement member 204. The displacement member 204 is connected to a threaded portion of the lead screw 203 in a threaded manner. The displacement member 204 is fixedly connected to a main shaft 6. The lead screw 203 is rotatable to drive the displacement member 204 to move axially, which in turn drives the main shaft 6 to move axially, and the main shaft 6 can further control the effector to perform closing and stapling operations. The driven gear 202 is movably sleeved on the lead screw 203, and can drive the lead screw 203 to rotate synchronously through a spline arranged at a central portion of the driven gear.

Furthermore, the lead screw 203 is sleeved thereon with a spring 205 that abuts against a front end surface of the driven gear 202. The spring 205 can apply a driving force to the driven gear 202 all the time to drive the driven gear 202 to move axially toward a tail end of the lead screw 203. In some feasible embodiments, the spring 205 has a pre-shrinkage, such that the elastic force of the spring 205 can always apply a driving force to the driven gear 202 to drive it to move toward the tail end of the lead screw 203. In other feasible embodiments, the spring 205 may also be replaced by a tension spring, which is arranged at a rear end surface of the driven gear 202 to apply a pulling force to the driven gear 202 such that the driven gear 202 is moved toward the tail end of the lead screw 203.

As shown in FIG. 1 and FIG. 2, a top end of the first limit member 401 is fixedly connected with the connecting block 4, and a fixing block 206 is arranged at the tail end of the lead screw 203. In an initial state, a bottom potion of the first limit member 401 is sandwiched between the rear end surface of the driven gear 202 and a front end surface of the fixing block 206 to limit axial position of the driven gear 202, such that the driven gear 202 and the driving gear 201 are always in meshing; in a second state, the first limit member 401 moves radially so that its bottom portion is separated from the driven gear 202 and the fixing block 206, meanwhile, the driven gear 202 is driven by the spring 205 to move axially and is separated from the driving gear 201. In some feasible embodiments, the bottom potion of the first limit member 401 is matched with an outer contour of the lead screw 203, such that the first limit member 401 fits with the lead screw 203, ensuring the stability of the first limit member 401 in normal use.

Furthermore, a second manual member 7 is embedded in the end cover 101. The second manual member 7 is fixedly connected to the tail end of the lead screw 203, and rotation of the second manual member 7 can drive the lead screw 203 to rotate synchronously. In some feasible embodiments, the second manual member 7 may be rod-shaped, and a proximal end surface of the second manual member 7 is concave to form a recess such that a tool may be inserted therein to drive the second manual member 7 to rotate.

In this scheme, the arrangements of the first manual member 5 and the second manual member 7 allows the stapler to be operated in a manual mode instead of an electric mode during surgery, in an emergency situation such as unexpected low battery or failure of the first drive motor 200 that causes failure of the electric mode of the firing control assembly, to continue to complete the surgery through manual operation in the manual mode.

The working principle may be described as follows: first, the first manual member 5 is rotated, rotation of the first manual member 5 in turn drives the connecting block 4 to move radially, such that radial movement of the first limit member 401 synchronously drives the connecting block 4 to move radially to release the limit on the driven gear 202 in the axial direction, therefore the driven gear 202 is driven by the spring 205 to move toward the tail end of the lead screw 203 and is separated from the driving gear 201, and thus the power transmission from the first drive motor 200 to the lead screw 203 is discontinued. Then, the second manual member 7 is rotated to drive the lead screw 203 to continue to rotate, which transmits power to the effector such that the effector continues to perform the closing operation to staple the tissue, so as to ensure the smooth progress of the operation, avoid repeated actions of a secondary operation, and ensure completion rate of the operation. In this solution, the battery is accommodated between the end cover 101 and the housing 1. In case of failure, the battery needs to be removed first, and then the first manual member 5 and the second manual member 7 may be driven to rotate through corresponding connecting pieces that match with them. The connecting pieces may be any components that can be connected to the first manual member 5 and the second manual member 7 and are easy to operate manually, which is not focus of this solution and will not be elaborated herein.

The swing control assembly is shown in FIGS. 2, 3 and 5. A through hole is provided at a connection of the driving block 301 and the driving rod 303. A top portion of the plug-in connector 302 is engaged in a top groove 3011 of the driving block 301, and a rod portion of the plug-in connector 302 is inserted in the through hole to connect the driving block 301 and the driving rod 303. A side of the plug-in connector 302 is connected to the second limit member 402.

Specifically, a bottom end of the side of the plug-in connector 302 has an outwardly protruding foot 3021, and the second limiting member 402 has an elongated slot 4021 arranged parallel to the driving rod 303. The foot 3021 is slidably arranged in the elongated slot 4021 such that the plug-in connector 302 is movable axially relative to the second limiting member 402. At the same time, the arrangement in which the second limiting member 402 is connected with the plug-in connector 302 enables the second limiting member 402 to always limit the plug-in connector 302 in the radial direction, and can control the connection between the driving block 301 and the driving rod 303 by radially moving the plug-in connector 302, thereby controlling the transmission connection between the second drive motor 300 and the effector.

The working principle of the swing control assembly controlling the effector to swing may be as follows: a distal end of the driving rod 303 is hook-shaped, and is engaged with a connecting ring 304 slidably disposed on the main shaft 6, and is further connected to a transmission rod 305 that is fixedly connected to a side of the connecting ring 304 through the connecting ring 304. The second drive motor 300 drives the driving block 301 to move axially by rotating its motor shaft, the driving block 301 in turn drives the driving rod 303 and the transmission rod 305 to move synchronously axially to control the effector to swing and move to the surgical site.

During surgery, the effector usually needs to swing to accurately align with the surgical site, in case that the second drive motor 300 fails due to low battery or other reasons, it generally needs to adjust the effector to its neutral position such that it can be withdrawn safely. Therefore, the transmission connection between the second drive motor 300 and the effector needs to be discontinued.

In particular, as shown in FIGS. 1 to 7, the second limit member 402 is an elongated extension plate, which is configured to connect the connecting block 4 and the plug-in connector 302. A proximal end of the second limit member 402 is provided with an inclined extending driving waist-shaped hole 4022, and a side wall of the connecting block 4 is fixedly disposed with a driving pin 403, which is slidably engaged in the driving waist-shaped hole 4022. The driving pin 403 moves radially synchronously with the connecting block 4, and radial movement of the driving pin drives the second limit member 402 to move radially synchronously. In some feasible embodiments, the driving waist-shaped hole 4022 may be inclined toward the tail end of the second limit member 402 at an angle of 45 degrees.

In order to ensure consistency of synchronous movement of the distal end and the proximal end of the second limit member 402, the second limit member 402 is further provided thereon with at least one driven waist-shaped hole 4023 arranged perpendicular to the driving waist-shaped hole 4022, and a positioning pin 404 is slidably engaged in the driven waist-shaped hole 4023. The positioning pin 404 is fixedly disposed on the housing 1. In the illustrated embodiment, two driven waist-shaped holes 4023 are provided on the second limit member 402. The arrangement in which the driven waist-shaped hole 4023 is perpendicular to the driving waist-shaped hole 4022 enables the connecting block 4 to drive the second limit member 402 to synchronously move radially as a whole.

In the present disclosure, the first manual member 5 is provided to rotate and drive the connecting block 4 to move radially. The connecting block 4 drives the first limit member 401 and the second limit member 402 to move radially synchronously to simultaneously release the power transmissions between the first drive motor 200, the second drive motor 300 and the effector, such that, in the failure mode, the effector can return to its neutral position for safe exit, and rotation of the second manual member 7 may be used to replace the first drive motor 200 to transmit power to the effector so as to continue to complete the anastomosis of the tissues and ensure smooth progress of the operation. The configuration is simple and the manufacturing cost is low, which is conducive to popularization and use.

It should be understood that, although description is made herein according to the embodiments, it does not mean that each embodiment contains only one independent technical solution. Such kind of description is provided herein only for the sake of clarity. Those skilled in the art should consider the specification as a whole. Technical solutions according to various embodiments may also be appropriately combined to form other embodiment(s) that can be understood by those skilled in the art.

The series of detailed descriptions listed above are only descriptions of feasible embodiments of the present disclosure. They are not intended to limit the protection scope of the present disclosure. Any equivalent implementation methods or changes that do not deviate from the technical spirit of the present disclosure should fall within the protection scope of the present disclosure.

## Claims

1. A stapler that is manually operable in case of failure in electric mode, comprising:
a housing;
an effector disposed at a front end of the housing;
a firing control assembly arranged in the housing for driving the effector to perform closing and anastomosing operations; and
a swing control assembly for driving the effector to swing, wherein
the firing control assembly comprises a first drive motor and a gear set, wherein the gear set comprises a driving gear disposed on a motor shaft of the first drive motor, and a driven gear configured to mesh with the driving gear and transmit power to the effector;
the swing control assembly comprises a second drive motor and a transmission assembly, wherein the transmission assembly comprises a driving block threadedly connected on a motor shaft of the second drive motor, and a driving rod that is configured to receive an axial driving force of the driving block through a plug-in connector and transmits the driving force to the effector;
**characterized by** further comprising a first manual member and a connecting block, wherein a proximal side of the connecting block is configured to engage with the first manual member and be driven by the first manual member to displace radially, and a distal side of the connecting block is disposed with a first limiting member and a second limiting member that synchronously displace radially with the connecting block;
the first limiting member is configured to release axial limitation on the driven gear by means of radial displacement of the first limiting member, such that the driven gear can be forced to displace axially to disengage from the driving gear, thereby cutting off power transmission between the first drive motor and the effector; and at the same time, the second limit member is configured to drive the plug-in connector to move synchronously radially by means of radial displacement of the second limit member, to disengage the driving block from the driving rod, thereby cutting off power transmission between the second drive motor and the effector.

2. The stapler that is manually operable in case of failure in electric mode according to claim 1, **characterized in that**, the first manual member is embedded in an end cover, and a proximal end of the first manual member has a driving part that is configured to be manually driven to rotate, and an insertion rod is eccentrically arranged on a distal end surface of the first manual member, the insertion rod is plugged into the proximal side of the connecting block to convert rotational movement of the first manual member into radial movement of the connecting block.

3. The stapler that is manually operable in case of failure in electric mode according to claim 2, **characterized in that**, the firing control assembly further comprises a lead screw and a displacement member, wherein the displacement member is threadedly connected on a threaded portion of the lead screw, the displacement member is fixedly connected to a main shaft; the lead screw is configured to drive the displacement member to drive the main shaft to move axially by means of rotation of the lead screw; and the driven gear is movably sleeved on the lead screw, and can drive the lead screw to rotate synchronously through a spline arranged at a central portion of the driven gear.

4. The stapler that is manually operable in case of failure in electric mode according to claim 3, **characterized in that**, a spring is sleeved on the lead screw and abuts against a distal end surface of the driven gear, and the spring always applies a driving force to the driven gear to drive the driven gear to move axially toward a tail end of the lead screw.

5. The stapler that is manually operable in case of failure in electric mode according to claim 4, **characterized in that**, a top portion of the first limit member is fixedly connected to the connecting block, and the tail end of the lead screw is disposed with a fixing block; in an initial state, a bottom portion of the first limit member is engaged between a proximal end surface of the driven gear and a distal end surface of the fixing block to limit axial position of the driven gear such that the driven gear and the driving gear remain in meshing; and in a second state, the first limit member moves radially such that its bottom portion is separated from the driven gear and the fixing block, and at the same time, the driven gear is driven by the spring to move axially and is separated from the driving gear.

6. The stapler that is manually operable in case of failure in electric mode according to claim 3, **characterized in that**, a second manual member is embedded in the end cover, wherein the second manual member is fixedly connected to a tail end of the lead screw, and can drive the lead screw to rotate synchronously by means of rotation of the the second manual member.

7. The stapler that is manually operable in case of failure in electric mode according to claim 1, **characterized in that** a through hole is provided at a connection of the driving block and the driving rod; a top portion of the plug-in connector is engaged in a top groove of the driving block, a rod portion of the plug-in connector is inserted in the through hole to connect the driving block and the driving rod, and a side of the plug-in connector is connected to the second limit member.

8. The stapler that is manually operable in case of failure in electric mode according to claim 7, **characterized in that**, a bottom end of the side of the plug-in connector has an outwardly protruding foot, the second limiting member has an elongated groove that is arranged parallel to the driving rod, and the foot is slidably arranged in the elongated groove such that the plug-in connector can move axially relative to the second limiting member.

9. The stapler that is manually operable in case of failure in electric mode according to claim 8, **characterized in that**, the second limiting member is an elongated extension plate, and a proximal end of the second limiting member is disposed with an inclined extending driving waist-shaped hole; a driving pin is fixedly arranged on a side wall of the connecting block, the driving pin is slidably arranged in the driving waist-shaped hole, and can drive the second limiting member to move radially synchronously by means of radial movement of the driving pin.

10. The stapler that is manually operable in case of failure in electric mode according to claim 9, **characterized in that**, the second limiting member is further provided thereon with at least one driven waist-shaped hole that is arranged perpendicular to the driving waist-shaped hole; a positioning pin is slidingly arranged in the driven waist-shaped hole; and the positioning pin is fixedly arranged on the housing.
